(19) Europäisches Patentamt
European Patent Office
Office européen des brevets

(11) Publication number: **0 069 584**
**B1**

(12) # EUROPEAN PATENT SPECIFICATION

(45) Date of publication of patent specification: **05.06.85**

(51) Int. Cl.$^4$: **G 03 C 7/32,** C 07 C 143/72, C 09 B 17/02, C 07 D 241/46

(21) Application number: **82303536.5**

(22) Date of filing: **06.07.82**

(54) **Color-forming sulfonamidodiphenylamines, photographic elements containing them and corresponding phenazine dyes.**

(30) Priority: **06.07.81 US 280673**
**06.07.81 US 280628**

(43) Date of publication of application:
**12.01.83 Bulletin 83/02**

(45) Publication of the grant of the patent:
**05.06.85 Bulletin 85/23**

(84) Designated Contracting States:
**DE FR GB**

(56) References cited:
**EP-A-0 029 546**
**DE-B-1 197 462**
**US-A-3 482 971**

**RESEARCH DISCLOSURE, no. 210, October 1981, no. 21003, pages 372-373, Havant, Hampshire (GB); "Photographic materials and processes comprising color-forming sulfonamidodiphenylamine dye precursors and corresponding phenazine dyes"**

(73) Proprietor: **EASTMAN KODAK COMPANY**
**343 State Street**
**Rochester New York 14650 (US)**

(72) Inventor: **Gabrielsen, Rolf Steven**
**1729 Empire Boulevard-Apt.66**
**Webster New York 14580 (US)**
Inventor: **Graham, Patricia Ann**
**6594 Fisher Road**
**Williamson New York (US)**
Inventor: **Klijanowicz, James Edward**
**7 Millstone Court**
**Pittsford New York 14534 (US)**

(74) Representative: **Baron, Paul Alexander Clifford et al**
**Kodak Limited Patent Department Headstone Drive**
**Harrow Middlesex HA1 4TY (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 069 584 B1

## Description

This invention relates to a color-forming sulfonamidodiphenylamine dye precursor that, in oxidized form, intramolecularly reacts to produce a sulfonamido-substituted phenazine dye. It also relates to phenazine dyes formed from such sulfonamidodiphenylamines and photographic elements containing them.

Photographic materials for producing silver and dye images are well known. It has been desirable to provide alternative means for producing a dye image, especially a dye image that enhances a silver image, other than by coupling reactions. The oxidation of leuco dyes to form an image is described in U.S. Patent 3,938,995.

Reducing agents are known that autoreact intramolecularly to form a heterocyclic ring. Such reducing agents are described in, for example, U.S. Patent 3,482,971 and U.S. Patent 3,622,603. The reducing agents in these patents are formed by means of a hydroxy (OH) group or amino group in the position para to an —NH— group in the compounds. The compounds of the present invention have no such hydroxy group or amino group. The compounds of U.S. Patent 3,482,971 and U.S. Patent 3,622,603 are not disclosed as color-providing materials, but rather as reducing agents and scavengers for oxidized reducing agents.

According to the present invention there is provided a color-forming sulfonamidodiphenylamine dye precursor compound having sulfonamido groups in a para position and one or two of the ortho positions in relation to the —NH— moiety of the sulfonamidodiphenylamine and wherein the sulfonamidodiphenylamine is capable, in oxidized form, of reacting intramolecularly to produce a sulfonamido substituted phenazine dye.

Preferably, the benzene ring of the diphenylamine which does not contain the parasulfonamido group contains an alkoxy, amino, mono- or di-alkylamino group in its para position.

The color-forming sulfonamidodiphenylamine dye precursor is especially useful in an imaging material, such as a photographic silver halide material, to form a phenazine dye image by a cross-oxidation reaction. The color-forming sulfonamidodiphenylamine dye precursor does not have reducing properties that adversely affect image formation. In addition, the corresponding phenazine dye is useful as an image dye alone or as an image dye that enhances a metal image, such as a silver image, in an imaging material. The phenazine dye is also useful as a colorant for textiles, varnishes, waxes, leather, plastics, paper and paints.

Combinations of color-forming sulfonamidodiphenylamine dye precursors are also useful, if desired. A preferred class of color-forming sulfonamidodiphenylamine dye precursors according to the invention are represented by the following formula wherein all alkyl, aryl and alkoxy groups are optionally substituted:

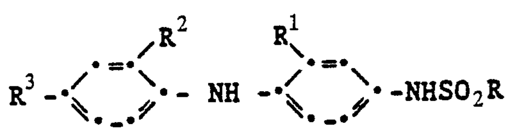

wherein:

R is alkyl containing 1 to 20 carbon atoms, such as methyl, ethyl, propyl, butyl, decyl, eicosyl, benzyl and xylyl; or aryl containing 6 to 20 carbon atoms, such as phenyl, para-tolyl or 2,4,6-triiso propylphenyl;

$R^1$ and $R^2$ are individually hydrogen, alkyl containing 1 to 4 carbon atoms, such as methyl, ethyl, propyl or butyl; or $—NHSO_2R^4$; and at least one of $R^1$ and $R^2$ is $—NHSO_2R^4$;

$R^3$ is alkoxy containing 1 to 20 carbon atoms such as methoxy, ethoxy, propoxy, or butoxy; alkyl containing 1 to 20 carbon atoms, such as methyl, ethyl, propyl, decyl or eicosyl; or

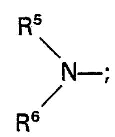

$R^4$ is alkyl containing 1 to 20 carbon atoms, such as methyl, ethyl, propyl, decyl, eicosyl, benzyl or xylyl; or aryl containing 6 to 20 carbon atoms, such as phenyl, para-tolyl ord 2,4,6-triiso propylphenyl;

$R^5$ is hydrogen or alkyl containing 1 to 20 carbon atoms, such as methyl, ethyl, propyl, decyl or eicosyl; and

$R^6$ is alkyl containing 1 to 20 carbon atoms, such as methyl, ethyl, propyl, butyl, decyl or eicosyl.

The terms "alkyl", "aryl" and "alkoxy" herein include unsubstituted and substituted groups. As will be readily understood, these groups may be substituted by groups which do not adversely affect the desired properties of the sulfonamidodiphenylamines or the corresponding phenazine dyes. Examples of useful substituted alkyl groups include alkyl substituted by alkoxy, hydroxyalkoxy, carboxamido or methanesulfonamido groups. Examples of substituted aryl include methoxyphenyl, 2,4,6-triisopropylphenyl and tolyl.

An optimum color-forming sulfonamidodiphenylamine dye precursor according to the invention will depend upon such factors as the intended use, the desired image, the particular imaging material,

processing steps and conditions in forming an image in the imaging material, the particular photosensitive silver halide and the imaging material containing the sulfonamidodiphenylamine dye precursor, and other components in the imaging material.

An especially useful color-forming sulfonamidodiphenylamine dye precursor is one wherein R in the noted formula is 2,4,6-triisopropylphenyl:

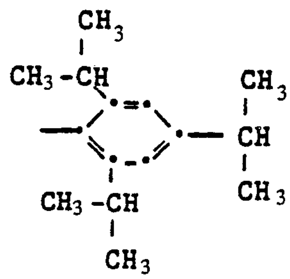

A 2,4,6-triisopropylphenyl group is also designated herein as follows:

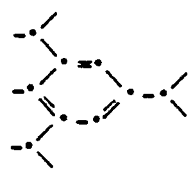

This group helps provide increased light stability to the resulting phenazine dye upon processing of the color-forming sulfonamidodiphenylamine dye precursor in a photographic silver halide material.

Examples of useful color-forming sulfonamidodiphenylamine dye precursors include:

2′,4′-Bismethanesulfonamido-4-diethylaminodiphenylamine represented by the formula:

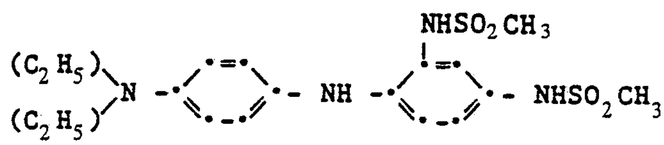

2′-Methanesulfonamido-4-(2,4,6-triisopropyl)benzenesulfonamido-2-methyl-4-N-(2-methanesulfonamidoethyl)ethylamido diphenylamine represented by the formula:

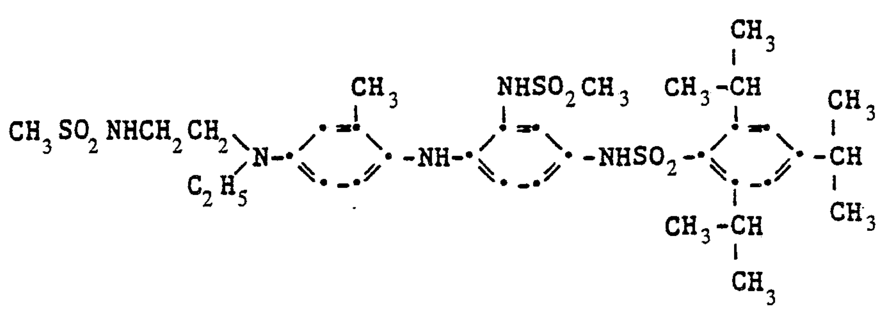

2′-Methanesulfonamido-4-(2,4,6-triisopropyl)benzenesulfonamido-4-(hydroxytrisethoxy)-diphenylamine represented by the formula:

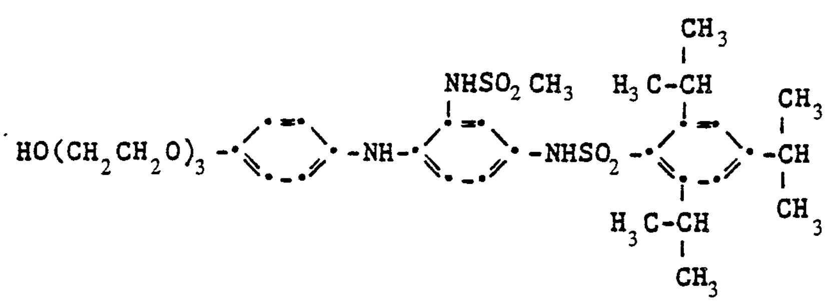

4-N-(2-Methanesulfonamidoethyl)ethylamino-2-methyl-2′,4′-bis(2,4,6-triisopropyl)benzenesulfonamido diphenylamine represented by the formula:

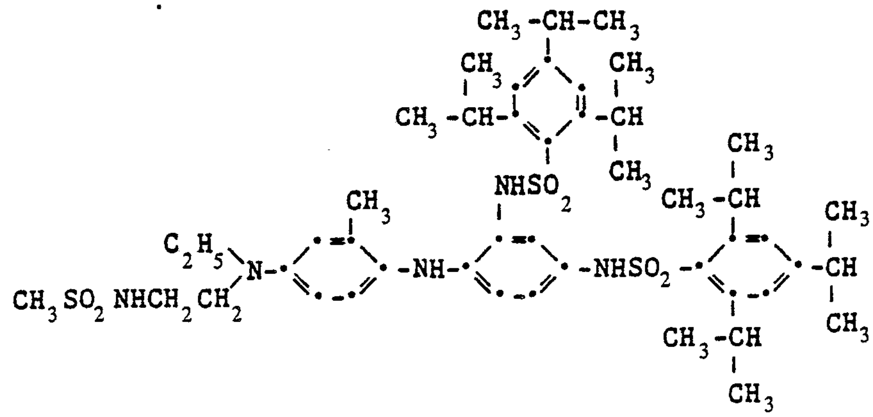

2,4′-Bismethanesulfonamido-4-N,N-diethylaminodiphenylamine represented by the formula:

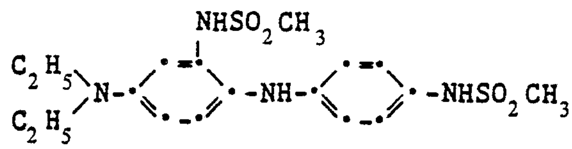

4-*n*-Hexyloxy-2′-methanesulfonamido-4′-(2,4,6-triisopropyl)benzenesulfonamido diphenylamine represented by the formula:

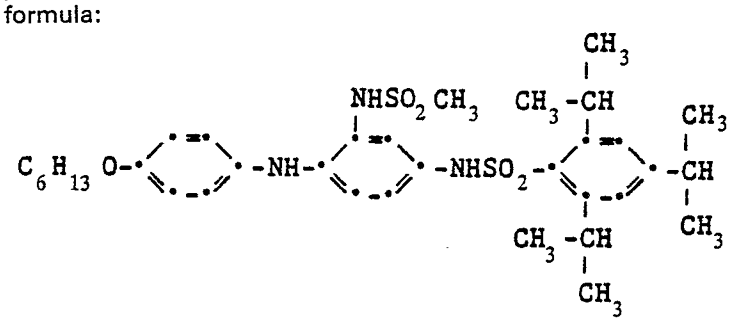

4-Methoxy-2′-methanesulfonamido-4′-(2,4,6-triisopropyl)benzenesulfonamido diphenylamine represented by the formula:

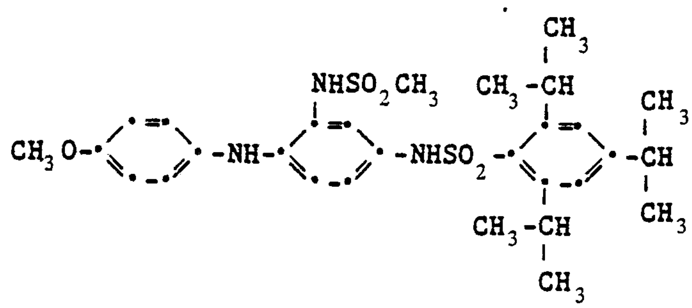

Each of the color-forming sulfonamidodiphenylamine dye precursors forms a corresponding phenazine dye by intra-molecular reaction.

The sulfonamidodiphenylamines may be prepared by methods known in themselves. A preferred method of preparing color-forming sulfonamidodiphenylamines comprises the steps:

(I) reacting (A) represented by the formula:

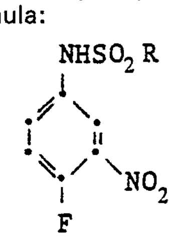

with

(B) represented by the formula:

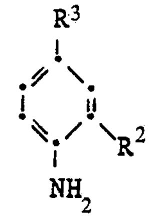

wherein

R, $R^2$ and $R^3$ are as defined, to produce a nitro-substituted sulfonamidodiphenylamine;

(II) reducing the resulting nitro substituted sulfonamidodiphenylamine from (I) by means of hydrogen in the presence of a suitable catalyst, preferably a Raney nickel catalyst; and, purifying the resulting intermediate amino-substituted sulfonamidodiphenylamine; then,

(III) dissolving the purified amino-substituted sulfonamidodiphenylamine from (II) in a suitable organic solvent, such as pyridine, and reacting the amino-substituted-sulfonamidodiphenylamine with a suitable sulfonyl chloride to form the color-forming sulfonamidodiphenylamine according to the invention.

An example of such a process of preparing a color-forming sulfonamidodiphenylamine dye precursor is the preparation of 2′,4′-bismethanesulfonamido-4-diethylaminodiphenylamine represented by the formula:

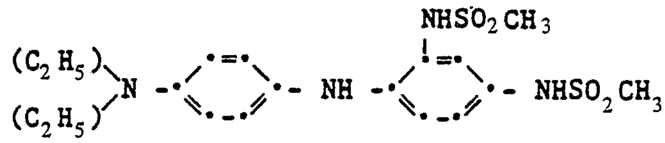

The process of preparing this color-forming sulfonamidodiphenylamine dye precursor comprises the steps:

(I) reacting a fluorine substituted compound represented by the formula:

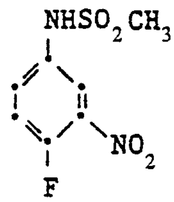

with an amine represented by the formula:

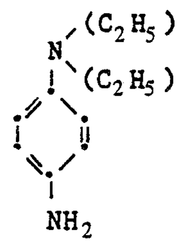

to release hydrogen fluoride (HF) and to form:

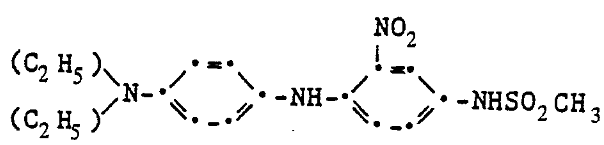

then,

(II) reducing the resulting nitro substituted sulfonamidodiphenylamine from (I) by means of hydrogen in the presence of suitable catalyst, such as a Raney nickel catalyst; and, purifying the resulting intermediate amino-substituted sulfonamidodiphenylamine; then,

(III) dissolving the purified, amino-substituted sulfonamidodiphenylamine from (II) in a suitable solvent, such as pyridine, and reacting the amino-substituted sulfonamidodiphenylamine with methanesulfonyl chloride to form the desired sulfonamidodiphenylamine according to the invention.

The described preparation of a color-forming sulfonamidodiphenylamine is generally carried out at atmospheric pressure. Step (I) in the process is generally carried out in the presence of a suitable solvent, such as alpha-picoline, 2,6-lutedine, pyridine, beta-picoline and gamma-picoline. This step involving the

production of the nitro substituted intermediate is generally carried out at reflux temperature under a nitrogen atmosphere to prevent adverse interference from oxygen or other components in the atmosphere. The nitro substituted sulfonamidodiphenylamine intermediate is generally separated and purified prior to its reduction in step (II) of the process. This separation and purification is carried out by conventional techniques in the organic synthesis art, such as recrystallization processes.

The reduction in step (II) of the process involves use of hydrogen in the presence of a suitable catalyst. This catalyst is preferably a Raney nickel catalyst. Other useful catalysts are palladium on carbon and platinum on carbon. The conditions under which the reduction is carried out in step (II) will depend upon such factors as the particular intermediate, the presence of a solvent, the particular catalyst, and the desired intermediate amino-substituted sulfonamidodiphenylamine.

In step (III) of the process, a sulfonyl chloride is suitable which provides a sulfonamido group in the ortho position in relation to the —NH— group of the color-forming sulfonamidodiphenylamine dye precursor. Many sulfonyl chlorides are useful. Examples of suitable sulfonyl chlorides include alkylsulfonyl chlorides, arylsulfonyl chlorides and alkarylsulfonyl chlorides containing up to 20 carbon atoms. The alkyl, aryl or alkaryl group of the sulfonyl chloride corresponds to the alkyl, aryl or alkaryl group for $R^1$ as defined for the color-forming sulfonamidodiphenylamine dye precursor. Examples of useful sulfonyl chlorides include methanesulfonyl chloride, triisopropylbenzenesulfonyl chloride, butanesulfonyl chloride and paratoluenesulfonyl chloride.

A solvent is suitable in step (III) of the process which is compatible with the intermediate purified amine substituted sulfonamidodiphenylamine from step (II). Pyridine is an especially useful solvent. Examples of other useful solvents include: alpha-picoline and dioxane with triethylamine. Selection of an optimum solvent will depend upon such factors as the particular intermediate amine substituted sulfonamidodiphenylamine, the particular sulfonyl chloride, reaction conditions and the desired color-forming sulfonamidodiphenylamine dye precursor.

The present invention also provides a phenazine dye having a sulfonamido group in the 2-position and preferably an alkoxy, amino or a mono- or dialkylamino group in the 8-position.

Many phenazine dyes are prepared from the color-forming sulfonamidodiphenylamine dye precursors according to the invention. Combinations of phenazine dyes are prepared, if desired. Examples of useful phenazine dyes prepared from the color-forming sulfonamidodiphenylamine dye precursors include those represented by the formula:

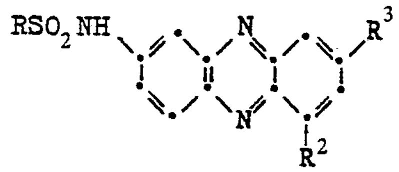

wherein:

R, $R^2$, and $R^3$ have the meanings given above.

An especially useful phenazine dye is one wherein R is 2,4,6-triisopropylphenyl. This group provides increased stability for the phenazine dye.

Selection of an optimum phenazine dye will depend upon such factors as the desired end use, such as the particular imaging material in which the phenazine dye is to be used, other components in the imaging material, and the desired color of the dye. Examples of useful phenazine dyes include:

2-Diethylamino-8-methanesulfonamido phenazine represented by the formula:

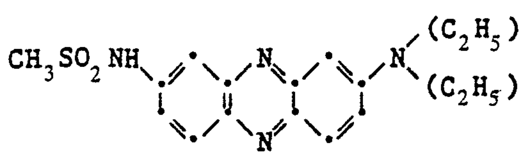

2-N-ethyl-N-(2-methanesulfonamidoethyl)ethylamino-4-methyl-8-(2,4,6-triisopropyl)benzenesulfonamido phenazine represented by the formula:

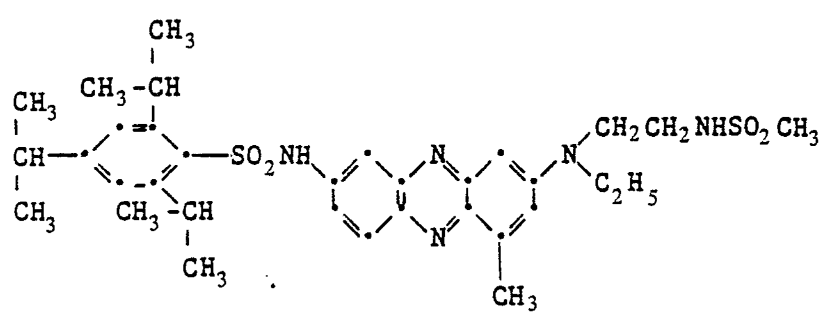

2-hydroxytrisethoxy-8-(2,4,6-triisopropyl)benzenesulfonamido phenazine represented by the formula:

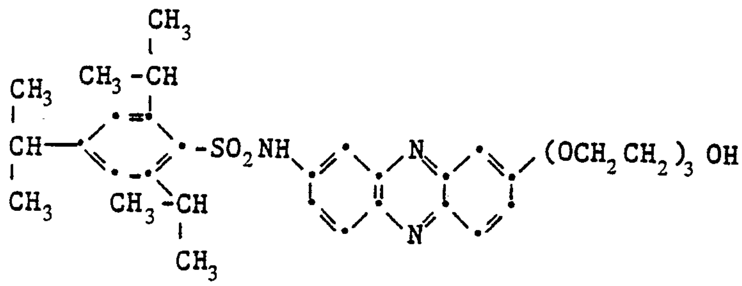

2-*n*-Hexyloxy-8-(2,4,6-triisopropyl)benzenesulfonamido phenazine represented by the formula:

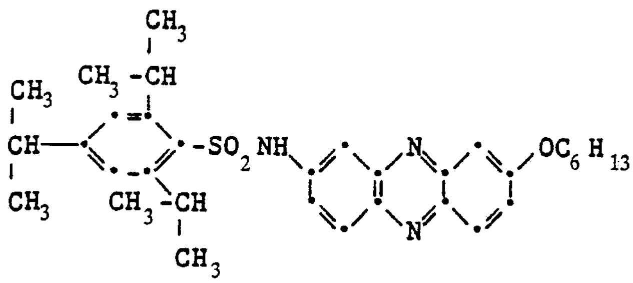

2-Methoxy-8-(2,4,6-triisopropyl)benzenesulfonamidophenazine represented by the formula:

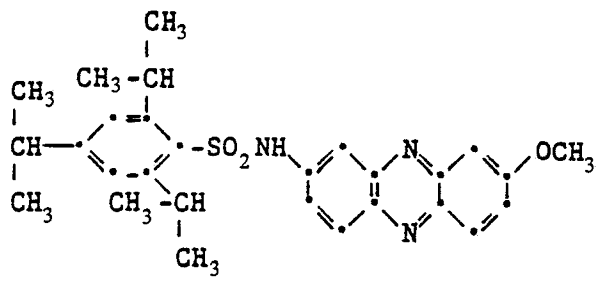

The hue of the phenazine dye will vary, depending upon such factors as the particular groups on the color-forming sulfonamidodiphenylamine dye precursor, processing conditions for preparation of the phenazine dye, other components in the composition containing the phenazine dye, such as dispersion solvents, and the physical form of the dye. In an imaging material, such as a photographic silver halide material, the color-forming sulfonamidodiphenylamine dye precursor is generally colorless or slightly colored prior to processing. Some of the starting color-forming sulfonamidodiphenylamine dye precursors have a slight yellow color in an imaging material. This slight color is not considered unacceptable in many imaging materials. In some imaging materials, such as in some photographic silver halide materials, the sulfonamidodiphenylamine dye precursor absorbs radiation in certain areas of the electro-magnetic spectrum which does not adversely affect the desired properties or the desired image of the phenazine dye formed upon processing.

The present invention further provides a photographic element comprising a support having thereon, in reactive association, a photographic silver salt and a color-forming dye precursor compound as described herein.

A process of preparing a phenazine dye comprises (I) oxidizing a color-forming sulfonamidodiphenylamine by means of an oxidizing agent, preferably the oxidized form of a 3-pyrazolidone reducing agent. This preparation and reaction is preferably carried out in an imaging material, such as a photographic silver halide material, in which the phenazine dye is formed imagewise.

With the present photographic silver salt materials, preferably silver halides, many cross-oxidizing reducing agents, also described as cross-oxidizing developing agents (COD), are useful for producing a phenazine dye. Any silver halide developing composition is useful for cross-oxidizing purposes, provided it comprises a major proportion of a cross-oxidizing reducing agent, which will cross-oxidize the sulfonamidodiphenylamine precursor to the desired phenazine dye. Such a cross-oxidizing reducing agent becomes oxidized during development of a silver halide photographic material by reducing exposed silver halide to silver metal. The oxidized reducing agent then cross-oxidizes the sulfonamidodiphenylamine dye precursor.

The cross-oxidizing reducing agent enables the sulfonamidodiphenylamine dye precursor to become

oxidized without the sulfonamidodiphenylamine dye precursor itself developing silver. Alternatively, the cross-oxidizing reducing agent is viewed as an electron transfer agent which shuttles electrons between the developing silver halide and the sulfonamidodiphenylamine dye precursor.

The general requirements for a suitable cross-oxidizing reducing agent in a photographic silver halide material are: (a) the reducing agent must have sufficient electrochemical potential under the conditions of use to develop exposed silver halide; (b) in its oxidized form, the reducing agent must be of such electrochemical potential to oxidize the sulfonamidodiphenylamine dye precursor; and, (c) in its oxidized form, the reducing agent must be sufficiently stable to undergo the redox reaction with the sulfonamidodiphenylamine dye precursor. If any of the conditions are not met, the reducing agent is not a suitable cross-oxidizing reducing agent for a silver halide photographic material. Whether a particular compound meets the requirements of a cross-oxidizing reducing agent depends upon the conditions under which the cross-oxidation reaction occurs. Other factors which influence the cross-oxidation reaction include the temperature of the process, the length of time required for cross-oxidation, and the pH of the composition in which cross-oxidation is to occur.

Examples of useful cross-oxidizing reducing agents include 3-pyrazolidone cross-oxidizing reducing agents. Preferred 3-pyrazolidone cross-oxidizing reducing agents include: 1-phenyl-3-pyrazolidone, 1-phenyl-4,4-dimethyl-3-pyrazolidone and 4-hydroxymethyl-4-methyl-1-phenyl-3-pyrazolidone. Such cross-oxidizing reducing agents are described in, for example, U.S. Patent 3,938,995. Combinations of cross-oxidizing reducing agents are also useful, if desired. Combinations of non-cross-oxidizing silver halide reducing agents and cross-oxidizing reducing agents are also useful if a minor proportion of the non-cross-oxidizing reducing agent is present, such as less than about 10 percent of the total of the reducing agents. Examples of combinations of a non-cross-oxidizing reducing agent and a cross-oxidizing reducing agent include: 4-hydroxymethyl-4-methyl-1-phenyl-3-pyrazolidone with a minor proportion of at least one of the non-cross-oxidizing reducing agents such as ascorbic acid, hydroquinone and pyrimidine derivatives. Selection of an optimum silver halide reducing agent or reducing agent combination will depend upon such factors as the desired image, the particular photosensitive silver halide, processing conditions, and the particular phenazine dye to be formed.

The photographic materials comprise a photographic metal salt, preferably a photographic silver salt such as photographic silver halide. It is essential that the photographic metal salt not adversely affect the desired imaging process, such as the intramolecular reaction that occurs in the photographic material. Examples of useful photographic silver halides are silver chloride, silver bromide, silver bromoiodide, silver chlorobromoiodide, silver iodide and mixtures thereof. The photographic silver halide is generally present in the photographic material in the form of an emulsion which is a dispersion of the photographic silver halide in a suitable binder. The photographic silver halide is present in a range of grain sizes from fine grain to coarse grain. The composition containing the photographic silver halide is prepared by any of the well known procedures in the photographic art, such as described in *Research Disclosure,* December 1978, Item No. 17643. The photographic silver halide material contains addenda commonly used in photographic silver halide materials, such as chemical sensitizers, brighteners, antifoggants, emulsion stabilizers, light absorbing or scattering materials, hardeners, coating aids, plasticizers, lubricants and antistatic materials, matting agents, development modifiers and other addenda described in *Research Disclosure,* December 1978, Item No. 17643.

A photographic silver halide composition containing the color-forming sulfonamidodiphenylamine dye precursor is generally spectrally sensitized by means of spectral sensitizing dyes, as described in, for example, the above *Research Disclosure* Item No. 17643. Dyes which are useful for spectrally sensitizing silver halide photographic materials are selected from such classes of spectral sensitizing dyes as polymethine dyes, which include the cyanines, complex cyanines and merocyanines (including tri, tetra and polynuclear cyanines and merocyanines), as well as oxonols, hemioxonols, styryls, merostyryls and streptocyanines. Combinations of spectral sensitizing dyes are also useful.

The described color-forming sulfonamidodiphenylamine dye precursor is in any suitable location in the photographic material which produces the desired phenazine dye upon processing. The color-forming sulfonamidodiphenylamine dye precursor should be in a location with respect to the photosensitive silver halide which produces the desired dye image and the desired silver image upon processing. If desired, a proportion of the color-forming sulfonamidodiphenylamine dye precursor is in a layer contiguous to the layer of the photographic element comprising photosensitive silver halide. The term "in reactive association" as used herein means that the photosensitive silver halide and the color-forming sulfonamidodiphenylamine dye precursor are in a location with respect to each other which enables the photographic material upon processing to produce a desired phenazine dye image and a desired silver image.

A range of concentrations of photographic silver halide is present in the photographic material. An optimum concentration of photographic silver halide will depend upon such factors as the desired image, processing conditions, particular dye precursor, other components in the photographic material, and particular cross-oxidizing reducing agent in the photographic material. A useful concentration of photographic silver halide in the photographic material is within the range of 1 mole to 10 moles of photographic silver halide per mole of dye precursor in the photographic material. The coverage of photographic silver halide may be less than otherwise might be useful, due to the enhancing properties of

the phenazine dye produced upon processing of the photographic material.

The silver halide developing agent or developing agent combination can be incorporated in the photographic material. Generally, the developing agent is most useful in a processing solution or it may be incorporated in the photographic element to produce a desired phenazine dye image and silver image.

The cross-oxidizing developing agent is useful in a range of concentrations in the photographic material or in a processing composition in which the photographic material is processed. A useful concentration of developing agent when the developing agent is present in the photographic material is within the range of 0.1 to 1.0 mole of developing agent per mole of color-forming sulfonamidodiphenylamine dye precursor in the photographic material. A useful concentration of developing agent in the processing solution for processing a photographic material containing a color-forming sulfonamidodiphenylamine dye precursor is within the range of 0.5 to 2 g of developing agent per liter of processing solution.

The term "developing agent" herein includes compounds which are developing agents and developing agent precursors. That is, those compounds are included which are not developing agents in the photographic material until a condition occurs, such as contact with a suitable activator for the photographic material.

The tone of the silver image and dye image produced in a photographic material varies, depending upon such factors as the silver morphology of the developed silver image, the covering power of the silver materials, the particular phenazine dye formed, the particular developing agent, processing conditions, and concentration of components. In photographic materials that provide a brown silver image, a phenazine dye produced from the described color-forming sulfonamidodiphenylamine dye precursor is especially useful which is complementary in hue to the silver image.

The photographic materials generally comprise a binder. Binders are useful alone or in combination in a photographic material. Useful binders include both naturally occurring substances such as proteins, for example, gelatin, gelatin derivatives, cellulose derivatives, polysaccharides such as dextran, gum arabic and the like; and synthetic polymeric materials such as water soluble polyvinyl compounds like polyvinyl pyrrolidone and acrylamide polymers.

If desired, the photographic elements may contain an overcoat layer and/or interlayer and/or subbing layer to provide desired properties. The overcoat layer, for example, increases resistance to abrasion and other markings on the element. The overcoat layer, interlayer or subbing layer contain, alone or in combination, vehicles and binders that are useful in the layer of the element containing the photosensitive silver halide. Gelatin is an especially useful binder.

A photographic element comprises a variety of supports. Useful supports include those which are resistant to adverse changes in structure due to processing conditions and which do not adversely affect the desired sensitometric properties of the photographic materials. Useful supports include cellulose ester, poly(vinyl acetal), poly(ethylene terephthalate) and polycarbonate films, as well as related films and resinous materials. Glass, paper, metal and the like supports are also useful. A flexible support is generally most useful.

Photographic materials are coated on a suitable support by procedures known in the photographic art. Such procedures include, for example, immersion or dip coating, roller coating, reverse roll coating, air knife coating, doctor blade coating, spray coating, extrusion coating, bead coating, stretch flow coating and curtain coating.

In preparing an imaging composition comprising the color-forming sulfonamidodiphenylamine dye precursor, a dispersion solvent, also described herein as a coupler solvent, is useful to produce a coating composition. Many suitable coupler solvents known in the photographic art are also useful for aiding the dispersion of the sulfonamidodiphenylamine dye precursor or the corresponding phenazine dye produced upon processing. Examples of useful coupler solvents include N-*n*-butylacetanilide, diethyl lauramide, di-*n*-butyl phthalate and 2,4-ditertiaryamylphenol. The color-forming sulfonamidodiphenylamine dye precursor is also usefully loaded into a latex or a non-solvent dispersion is prepared, if desired.

Photographic elements are usefully imagewise exposed by means of various forms of energy. The color-forming sulfonamidodiphenylamine dye precursor and the corresponding phenazine dye are relatively insensitive to light. However, the photosensitive component of the photographic element, such as photosensitive silver halide, is energy sensitive, especially photosensitive. The forms of energy which are useful to imagewise expose the photosensitive component include those to which photosensitive silver halide is sensitive and encompass such forms of energy as the ultraviolet, visible and infrared regions of the electromagnetic spectrum, as well as electron beam and beta radiation, gamma rays, X-rays, alpha particles, neutron radiation and other forms of corpuscular wave-like radiant energy, and either noncoherent (random phase) forms or coherent (in phase) forms, as produced by lasers. Imagewise exposure of the photographic material is monochromatic, orthochromatic or panchromatic, depending upon the spectral sensitization of the photosensitive silver halide. Imagewise exposure is generally for a sufficient time and intensity to produce a developable latent image in the photographic material.

The described photographic material containing the color-forming sulfonamidodiphenylamine dye precursor may be processed eithere in a process which produces a positive phenazine dye image, or in a process which produces a negative phenazine dye image with or without a silver image in each case. The photosensitive silver halide contained in the photographic material is processed following exposure to

form a visible image by associating the silver halide with an aqueous alkaline medium in the presence of a cross-oxidizing reducing agent contained in the medium or in the photographic material.

If a reversal phenazine dye image is desired in the photographic material, a process is most useful in which a non-cross-oxidizing developing composition is used for processing the exposed photographic material as a first development step. During this step, the exposed silver halide is reduced to elemental silver by the non-cross-oxidizing developing composition. The non-cross-oxidizing developing composition does not, when oxidized, oxidize the color-forming sulfonamidodiphenylamine dye precursor to its corresponding phenazine dye.

The non-cross-oxidizing developer compositions useful in this step are generally alkaline solutions comprising a non-cross-oxidizing reducing agent. Non-cross-oxidizing reducing agents are well known in the photographic art and include many silver halide reducing agents which will reduce exposed silver halide to silver, but will not cross-oxidize the color-forming sulfonamidodiphenylamine dye precursor to a corresponding phenazine dye.

In a second step of the process for forming a reversal phenazine dye image, fogging is accomplished by exposing the photographic element to light or by chemical fogging by means of fogging compositions known in the photographic art.

Following the described fogging step, a second silver halide developing step is carried out. This is carried out by means of a cross-oxidizing developing composition. It is in this step that the phenazine dye image is formed. Many silver halide developing compositions are useful in this step, provided that the developing composition cross-oxidizes the color-forming sulfonamidodiphenylamine dye precursor to a desired phenazine dye. Such silver halide developing compositions include aqueous alkaline solutions comprising a cross-oxidizing silver halide reducing agent, such as a 3-pyrazolidone cross-oxidizing silver halide reducing agent. The cross-oxidizing reducing agent becomes oxidized during development by reducing exposed or fogged silver halide to silver metal. The oxidized developer then cross-oxidizes the color-forming sulfonamidodiphenylamine dye precursor to produce the desired phenazine dye. A positive phenazine dye image is formed.

The color-forming sulfonamidodiphenylamine dye precursor is also usefully incorporated in a photothermographic material, such as a photothermographic silver halide material. Photothermographic materials in which the sulfonamidodiphenylamine dye precursor may be incorporated are described in, for example, *Research Disclosure,* June 1978, Item No. 17029. For example a dye image or dye and silver image is produced in a photothermographic material comprising, in binder, in reactive association: (a) a photographic silver halide such as a photographic silver halide emulsion; (b) a cross-oxidizing photographic silver halide developing agent; (c) an activating concentration of a suitable base-release agent; and (d) a color-forming sulfonamidodiphenylamine dye precursor. The photothermographic material is generally imagewise exposed to light to provide a developable latent image which is then developed by merely uniformly heating the photothermographic element to processing temperature, such as a temperature within the range of about 100°C to about 180°C. This also enables formation of the desired phenazine dye. Another form of photothermographic material comprises, in binder, in reactive association: (a) photographic silver halide, which is formed *in situ* or *ex situ;* (b) an oxidation reduction image forming combination comprising: (i) an organic metal salt oxidizing agent, especially a silver salt of a fatty acid, such as silver behenate, silver laurate or silver stearate, with (ii) an organic cross-oxidizing reducing agent for the oxidizing agent, such as a 3-pyrazolidone reducing agent; and (c) a color-forming sulfonamidodiphenylamine dye precursor as described. This photothermographic material is also imagewise exposed to light and then uniformly heated to provide a silver image and a phenazine dye image.

An advantage of the described phenazine dyes in a photographic material is that the dyes provide satisfactory stability to post-processing conditions and visible light exposure. A simple test is useful for establishing the degree of stability which is desired for a phenazine dye image produced from a color-forming sulfonamidodiphenylamine dye precursor. One such test is a test known in the photographic art in which the processed photographic element is exposed to a Simulated Average North American Skylight (SANS) with continuous 5,400 LUX of exposure at an average temperature of 21°C at 45 percent relative humidity. A comparison of the stability of the tested phenazine dye is then observed.

The color-forming sulfonamidodiphenylamine dye precursor may be incorporated in a photographic silver halide processing composition for producing a dye enhanced silver image. Such a processing composition comprises a cross-oxidizing photographic silver halide developing agent and the desired color-forming sulfonamidodiphenylamine dye precursor or a combination of such dye precursors. The photographic processing composition is, for example, a silver halide developing composition, a hardener composition or a stabilizing composition. The processing composition generally comprises a base or base-release agent, such as sodium hydroxide, trisodium phosphate and potassium carbonate. An example of a suitable photographic silver halide processing composition comprises a 3-pyrazolidone cross-oxidizing photographic silver halide developing agent and a color-forming sulfonamidodiphenylamine dye precursor comprising 2'-methanesulfonamido-4'-(2,4,6-triisopropylbenzene)sulfonamido-2-methyl-4-N-(2-methanesulfonamidoethyl)ethylamino diphenylamine.

The described photographic material may contain a silver halide developing agent. When a silver halide developing agent is present in the photographic material, a developed image is produced after imagewise exposure of the photographic material by contacting the material with an alkaline activator

solution which enables development of the exposed silver halide, as well as production of the desired phenazine dye.

Many alkaline activators are useful for developing an image in a photographic material comprising an incorporated silver halide developing agent. Useful alkaline activators include those that have been found useful in the photographic art, such as in stabilization processing. Examples of useful alkaline activators include sodium hydroxide, potassium hydroxide, trisodium phosphate $\cdot 12_2O$ (pH 12), sodium metaborate (pH 12), disodium phosphate and monosodium phosphate. The optimum alkaline activator will depend upon such factors as the desired image, the particular developing agent, processing conditions, and the particular color-forming sulfonamidodiphenylamine dye precursor. An especially useful alkaline activator comprises trisodium phosphate (pH 12).

The alkaline activator is useful in a range of concentrations. A generally useful concentration of alkaline activator is within the range of 10 to 50 g of alkaline activator per liter of activator solution which produces a pH in the range of 11 to 12. An optimum concentration of alkaline activator will depend upon such factors as the particular activator, the desired image, processing conditions, particular photosensitive silver halide and particular developing agent.

In preparing a phenazine dye from a color-forming sulfonamidodiphenylamine for compositions and uses that are non-photographic, other oxidizing agents are useful than the oxidized form of a cross-oxidizing reducing agent. Such oxidizing agents include, for example, organic peroxides, such as benzoyl peroxide and hydrogen peroxide, inorganic peroxides, ferricyanides, dichromates and permanganates.

The following examples are included for a further understanding of the invention.

Example 1

Preparation of 4-diethylamino-2',4'-bis(methanesulfonamido)diphenylamine

The following preparation was carried out:

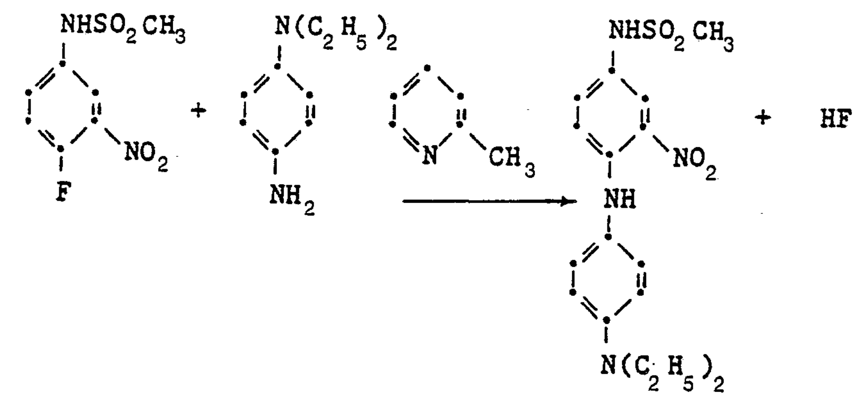

A solution of 42.1 g (0.18 mole) of 4-fluoro-3-nitro-methanesulfonamidobenzene and 29.5 g (0.18 mole) of N,N-diethyl-1,4-phenylenediamine in 250 ml of α-picoline (solvent) was refluxed overnight under nitrogen. The mixture was then poured over ice and, after the ice had melted, the mixture was filtered. The collected solid product was washed with water until clear washings were obtained, air dried, and recrystallized from ethyl acetate to provide 53.6 g of red solid identified as 4-diethylamino-3'-nitro-4'-methanesulfonamidodiphenylamine having a melting point of 168°C to 170°C. The compound was also identified by elemental analysis.

Then the following reaction sequence was carried out:

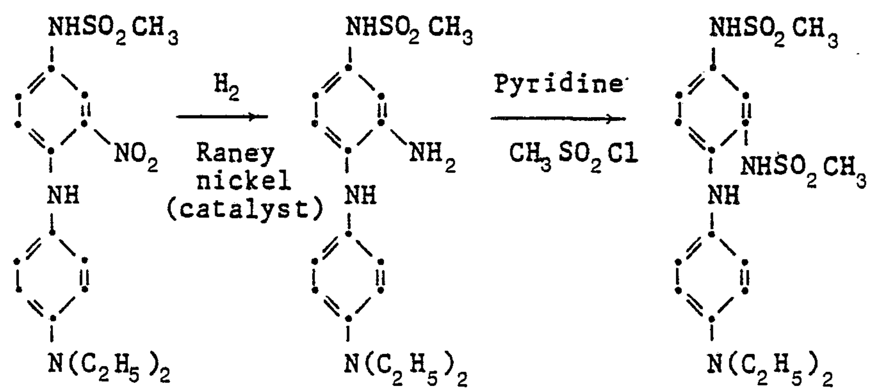


A solution of 20 g (0.053 mole) of 4-diethylamino-3'-nitro-4'-methanesulfonamidodiphenylamine in 400 ml of degassed tetrahydrofuran was reduced with hydrogen over Raney nickel catalyst at room

temperature at 275 kPa. The Raney nickel catalyst was removed by filtration, and the filtrate concentrated to dryness under nitrogen to protect it from air oxidation. A dark blue gum was obtained. This was dissolved in 250 ml of pyridine and treated with methanesulfonyl chloride.

After stirring overnight at room temperature, the solution was poured into 2 liters of water and extracted twice with ethyl acetate. The extracts were combined, washed 5 times with water and dried over magnesium sulfate. After concentration to dryness, a deep red gum which had the odor of pyridine was obtained. This was dissolved in 75 ml of toluene and titrated with about 125 ml of ligroin. A gum precipitated on the sides of the flask. After adding another 200 ml of ligroin with scratching and vigorous stirring, the gum began to solidify. A dark rose colored solid was collected that had the odor of pyridine. The solid was dissolved in 100 ml of ethyl acetate, washed with water and dried over magnesium sulfate. Concentration to dryness yielded a bright red glass. Thin layer chromatography indicated one major air sensitive component, some magenta dye and several minor impurities. The product was recrystallized twice from 100 ml of toluene containing a trace of ethyl acetate to produce a very pale pink solid having a melting point of 135°C to 137°C. Mass spectrographic analysis and nuclear magnetic resonance analysis confirmed the identity of the desired product as 4-diethylamino-2'-4'-bis(methanesulfonamido)diphenylamine. The product was also identified by elemental analysis.

The phenazine dye generated oxidatively from the sulfonamidodiphenylamine by means of $K_3Fe(CN)_6$ had a maximum absorption (in butyl acetate) of of 472 nm.

Examples 2 to 6

The following sulfonamidodiphenylamines were also prepared by methods analogous to that of Example 1.

Example 2

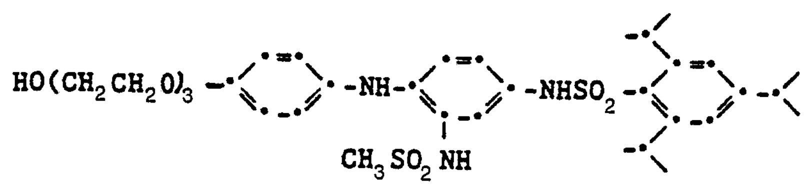

This compound had a melting point of 47 to 55°C.

Example 3

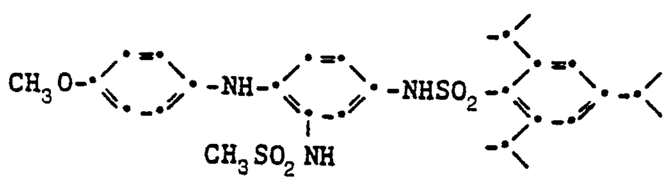

This compound had a melting point of 185 to 187°C.

Example 4

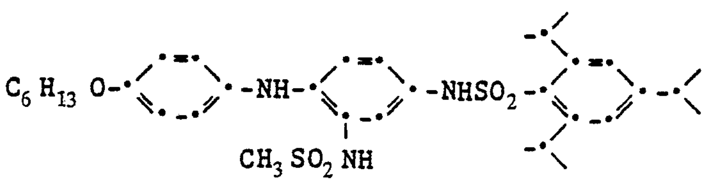

This compound had a melting point of 145 to 147°C.

Example 5

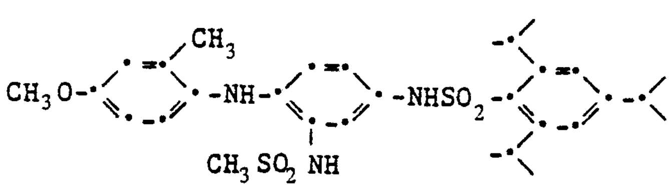

This compound had a melting point of 98 to 100°C.

Example 6

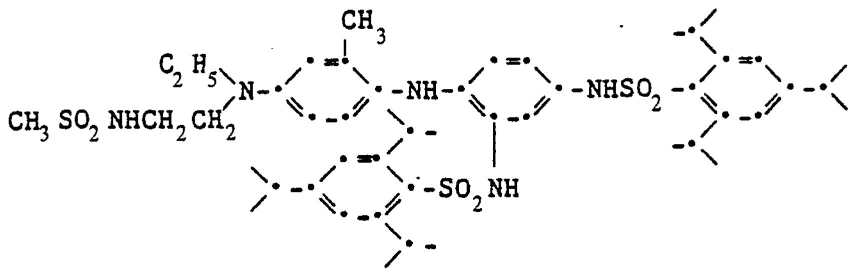

This compound had a melting point of 113 to 115°C.
Each of the above compounds was also identified by elemental analysis.

Examples 7 and 8

The corresponding phenazine dye was prepared from the sulfonamidodiphenylamine of Example 1 and from 4-methoxy-2′,4′-bis(methanesulfonamido)diphenylamine by the following procedure: A butyl acetate solution of the color-forming dye precursor was stirred rapidly with an aqueous solution of $K_3Fe(CN)_6$. After oxidation, the butyl acetate layer was washed with water and the solvent removed under reduced pressure to yield the desired phenazine dye. The following phenazine dyes were produced in this manner:

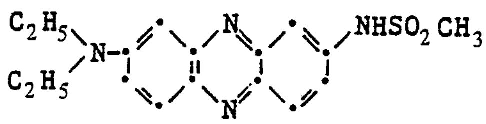

m.p. 216-217°C

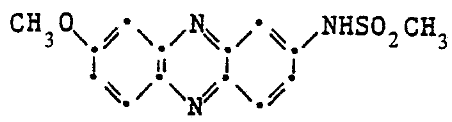

m.p. 287-289°C

Each of the phenazine dyes was identified by elemental analysis.

Example 9
Use in a Photographic Element

A photographic silver halide element was prepared by coating the following layer on a poly(ethylene terephthalate) film support:

| | |
|---|---|
| silver bromide | 9.72 $mg/dm^2$ |
| gelatin (binder) | 43.2 $mg/dm^2$ |
| bis(vinylsulfonylmethyl)ether (gelatin hardener) | 0.432 $mg/dm^2$ |
| dye precursor: | 13.6 $mg/dm^2$ |

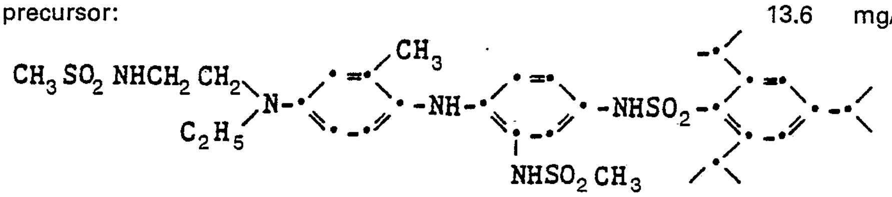

| | |
|---|---|
| di-*n*-butyl phthalate | (coupler 13.6 $mg/dm^2$ solvent) |

The resulting photosensitive silver halide layer was dried and then overcoated with a layer containing gelatin (10.8 $mg/dm^2$) and bis(vinylsulfonylmethyl)ether (hardener) (0.108 $mg/dm^2$). A strip of the resulting photographic film was imagewise exposed through a step tablet in a commercial sensitometer to produce

a developable image in the film. It was processed at 22°C by immersing the film in an agitated tank containing a developer composition. The developer composition contained the following:

| | |
|---|---|
| $Na_3PO_4 \cdot 12H_2O$ | 47.5 g |
| 4-hydroxymethyl-4-methyl-1-phenyl-3-pyrazolidone | 1.0 g |
| benzyl alcohol | 10.0 ml |
| distilled water to | 1 liter |

The film was immersed in the described developer for 30 seconds and then rinsed with water for 60 seconds. The film was then fixed by immersing the developed film in a fixer composition containing the following components:

| | |
|---|---|
| $Na_2S_2O_3 \cdot 5H_2O$ | 248 g |
| $Na_2CO_3 \cdot H_2O$ | 30 g |
| $NaHCO_3$ | 5 g |
| distilled water to | 1 liter |

The film was fixed for 30 seconds and then washed with water for 5 minutes. The film was then permitted to air dry.

The resulting developed silver plus dye image produced a maximum density in the first step of the step tablet of 1.2 and a minimum density in the eleventh step of the step tablet of 0.46. An orange phenazine dye image having a maximum absorption of 471 nm was generated during the described processing. This orange dye was a phenazine dye corresponding to the sulfonamidodiphenylamine dye precursor.

The phenazine dye image produced a maximum density of 0.79 to blue light.

Example 10

Use of Another Color-Forming Sulfonamidodiphenylamine Dye Precursor

The following composition was prepared and coated at a wet coating thickness of about 100 µm on gel-subbed poly(ethylene terephthalate) film support and dried at 37.8 to 40.6°C:

| | |
|---|---|
| Ethylene[bis-sulfonyl acetic acid] | 0.130 g |
| 1,3-Bis[2-S-(N,N′-ethyleneiso-thiourea)ethyl]urea·$2HNO_3$ | 0.078 g |
| 2,21-Dimethyl-7,16-dioxo-2,6,8,15,17,21-hexazadocosane | 0.060 g |
| 4-diethylamino-3,4′-bis(methanesulfonamido)diphenylamine | 0.062 g |
| 10% aqueous Surfactant 10G (para-isononylphenoxypolyglycidol, a trademark of and available from the Olin Corp., U.S.A.) | 0.2 ml |
| 10% aqueous gelatin | 2.5 ml |
| Water | 6.8 ml |
| Photographic Gelatino Emulsion (AgCl) | 0.21 g |
| pH adjusted to 4.0 with KOH | |

The resulting coating contained a silver coverage of 3.7 mg/$dm^2$ (34 mg/$ft^2$). A sample of the coating, after incubating for eleven days at 50% relative humidity and 37.8°C, was imagwise exposed for $10^{-3}$ second in a commercial sensitometer to produce a developable latent image in the element. The exposed

element was then processed by heating on a hot metal block at 160°C until a developed image was produced. A magenta dye-enhanced silver image was produced.

Example 11

The following gelatin-overcoated silver halide photographic element was prepared:

| | |
|---|---|
| Overcoat | |
| Gelatin | 10.8 mg/dm$^2$ |
| Bis(vinylsulfonylmethyl)ether | 0.11 mg/dm$^2$ |
| Photosensitive Layer | |
| AgBr, 0.8µm octahedral | 9.7 (as Ag°) |
| Gelatin | 43.2 |
| Bis(vinylsulfonylmethyl)ether | 0.43 |
| N-n-butylacetanilide | 15.9 |
| Color-former of the formula: | 15.9 |

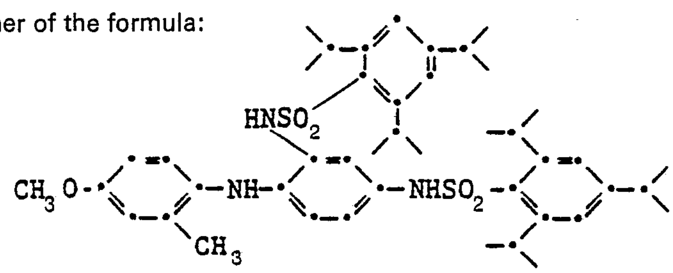

Samples of the coating were similarly exposed. One sample was developed in a MOP (4-hydroxymethyl-4-methyl-1-phenyl-3-pyrazolidone) developer* for 30 seconds at about 22°C and then fixed** for 30 seconds yielding a silver plus dye density ($\lambda_{max}$ at 470 nm) of 0.57. Another sample, similarly developed, was bleached*** until cleared yielding a dye-only density ($\lambda_{max}$ at 470 nm) of 0.36.

*pH—12 Phosphate buffered developer containing:

| | |
|---|---|
| $Na_3PO_4$ | 38.5 g |
| $Na_2HPO_4$ | 6.7 g |
| KBr | 1.0 g |
| Benzyl alcohol | 1% |
| MOP | 1.0 g |
| Water to one liter | |

** pH—10 (NaOH adjusted) fixing solution containing:

| | |
|---|---|
| $Na_2S_2O_3 \cdot 5H_2O$ | 248.0 g |
| $Na_2CO_3 \cdot H_2O$ | 30.0 g |
| $NaHCO_3$ | 5.0 g |
| Water to one liter | |

*** Bleach (similar to the composition below which is described on page 205 in *British Journal of Photography Annual 1979*):

| | |
|---|---|
| pH 5.9—6.1 | |
| NaFeEDTA | 100.0 g |
| KBr | 50.0 g |
| Ammonia (20%) | 6.0 ml |
| Water to one liter | |

Example 12

The photosensitive layer differed from Example 11 in that the N-*n*-butylacetanilide coverage was 9.9 mg/dm$^2$ and the color-forming dye precursor, represented by the following formula:

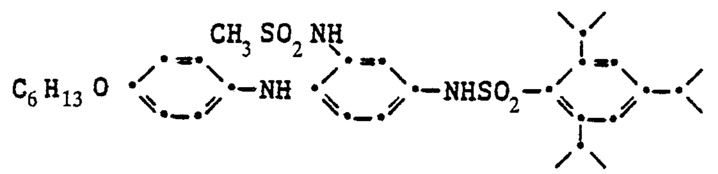

was substituted for the color-forming dye precursor of Example 11. The fixed sample had a silver plus dye density ($\lambda_{max}$ at 510 nm) of 0.68. The fixed and bleached sample had a dye density ($\lambda_{max}$ at 510 nm) of 0.53.

**Claims**

1. A color-forming sulfonamidodiphenylamine dye precursor compound having sulfonamido groups in a para position and in one or two of the ortho positions in relation to the —NH— moiety of the sulfonamidodiphenylamine and wherein the sulfonamidodiphenylamine is capable, in oxidized form, of reacting intramolecularly to produce a sulfonamido substituted phenazine dye.

2. A dye precursor compound according to claim 1 represented by the formula:

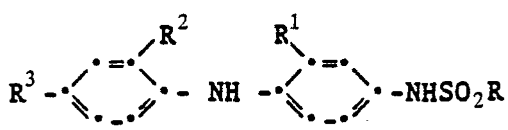

wherein:

R is alkyl containing 1 to 20 carbon atoms or aryl containing 6 to 20 carbon atoms;

$R^1$ and $R^2$ are individually hydrogen, alkyl containing 1 to 4 carbon atoms or $—NHSO_2R^4$; and at least one of $R^1$ and $R^2$ is $—NHSO_2R^4$;

$R^3$ is alkoxy containing 1 to 20 carbon atoms, alkyl containing 1 to 20 carbon atoms, or

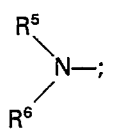

$R^4$ is alkyl containing 1 to 20 carbon atoms or aryl containing 6 to 20 carbon atoms;

$R^5$ is hydrogen or alkyl containing 1 to 20 carbon atoms; and

$R^6$ is alkyl containing 1 to 20 carbon atoms, all alkyl, aryl and alkoxy groups being optionally substituted.

3. A dye precursor compound according to claim 2 wherein R is 2,4,6-triisopropylphenyl.

4. A dye precursor compound according to claim 1 which has one of the formulae:

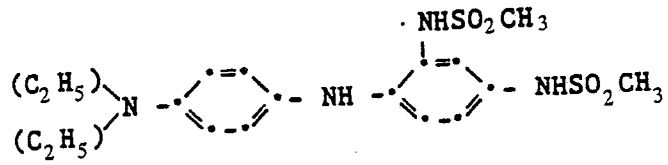

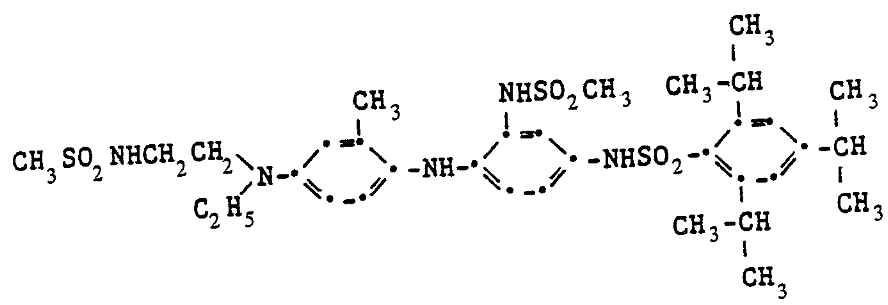

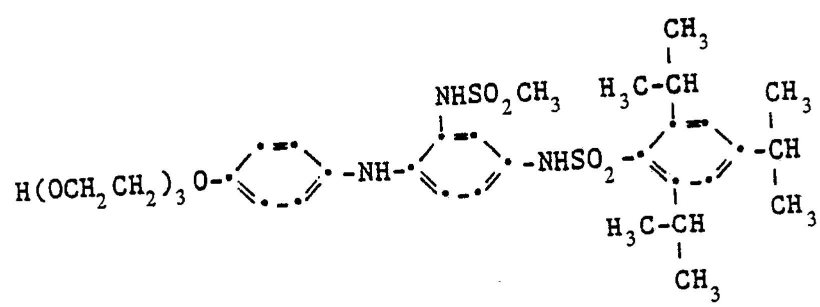

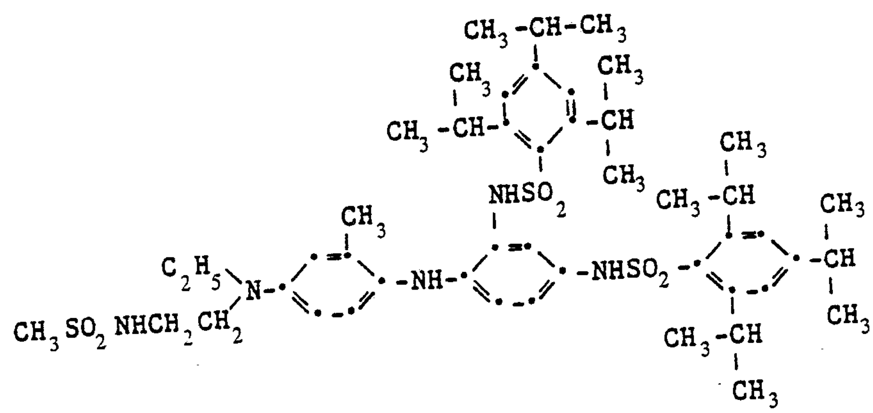

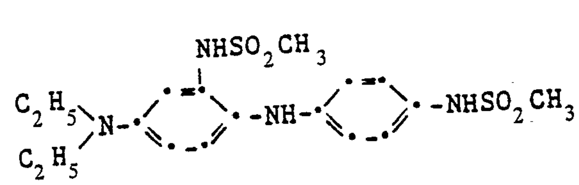

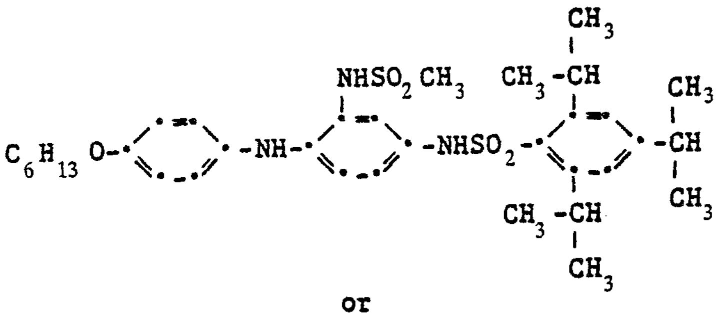

or

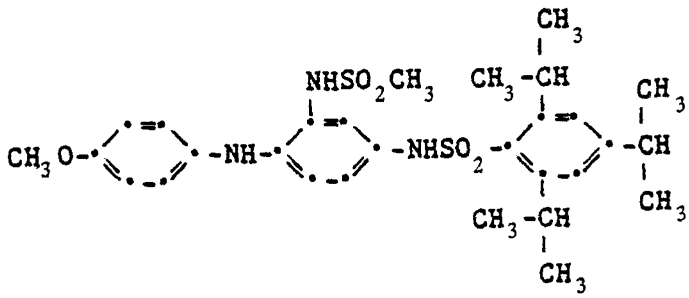

5. A photographic element comprising a support having thereon, in reactive association, a photographic silver salt and a dye precursor compound according to any of claims 1 to 4.

6. A photographic element according to claim 5 also comprising a binder.

7. A photographic element according to claim 5 or 6 wherein said photographic silver salt consists essentially of photographic silver halide.

8. A photographic element according to any of claims 5 to 7 containing a cross-oxidizing photographic silver halide developing agent.

9. A photographic element according to claim 8 wherein the photographic silver halide developing agent is a 3-pyrazolidone.

10. A photographic element according to any of claims 6 to 9 in which the binder is gelatin.

11. A phenazine dye having a sulfonamido group in the 2-position.

12. A phenazine dye according to claim 11 which further has an alkoxy, amino, mono- or di-alkylamino group in the 8-position.

13. A phenazine dye according to claim 11 or 12 represented by the formula:

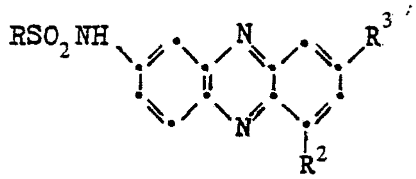

wherein:

R, $R^2$ and $R^3$ are as defined in claim 2.

14. A phenazine dye according to claim 13 wherein R is 2,4,6-triisopropylphenyl.

15. A phenazine dye according to claim 11 or 13 which has one of the formulae:

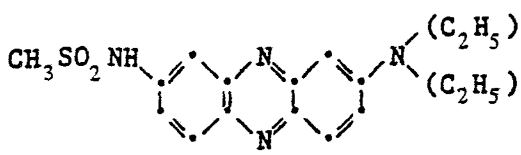

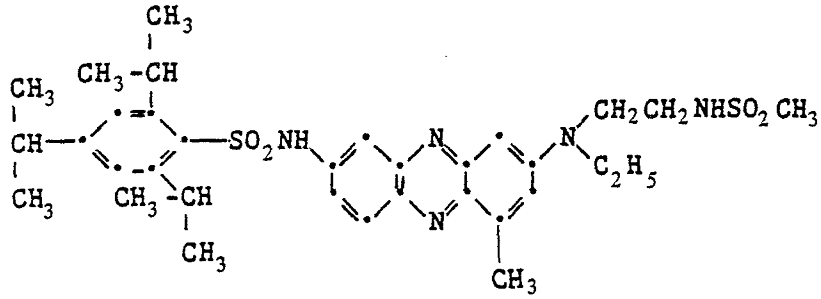

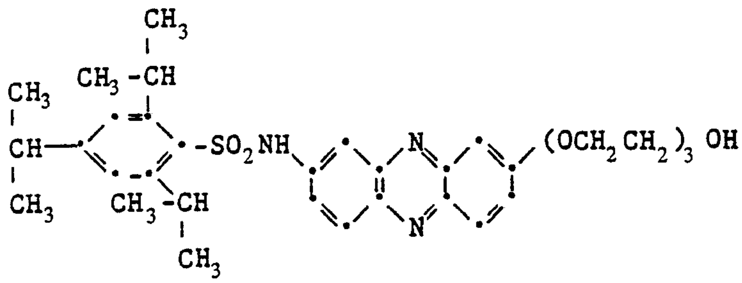

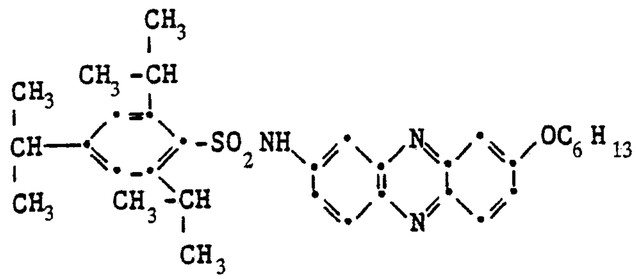

or

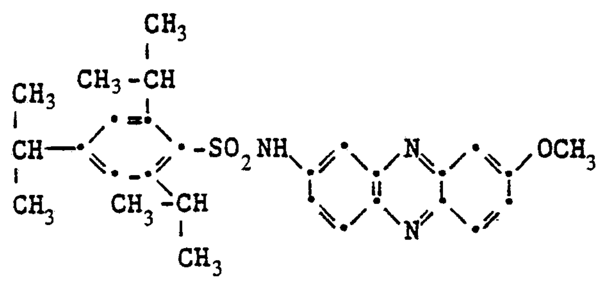

## Revendications

1. Composé chromogène précurseur de colorant du type sulfonamidodiphénylamine comprenant des groupes sulfonamido en position para et dans une ou deux des positions ortho par rapport au groupement —NH— de la sulfonamidodiphénylamine et dans lequel la sulfonamidodiphénylamine est capable, sous sa forme oxydée, de réagir intramoléculairement pour former un colorant phénazine sulfonamido substitué.

2. Composé précurseur de colorant conforme à la revendication 1 représenté par la formule suivante:

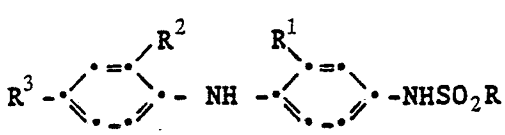

où:

R est alkyle contenant 1 à 20 atomes de carbone ou aryle contenant 6 à 20 atomes de carbone

$R^1$ et $R^2$ représentent chacun l'hydrogène, alkyle contenant 1 à 4 atomes de carbone ou $—NHSO_2R^4$, et au moins un des groupes $R^1$ et $R^2$ est $—NHSO_2R^4$,

$R^3$ est alkoxy contenant 1 à 20 atomes de carbone, alkyle contenant 1 à 20 atomes de carbone, ou

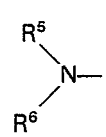

$R^4$ est alkyle contenant 1 à 20 atomes de carbone ou aryle contenant 6 à 20 atomes de carbone,

$R^5$ est l'hydrogène ou alkyle contenant 1 à 20 atomes de carbone, et,

$R^6$ est alkyle contenant 1 à 20 atomes de carbone, tous les groupes alkyle, aryle et alkoxy pouvant être substitués.

3. Composé précurseur de colorant, conforme à la revendication 2, dans lequel R est 2,4,6-triisopropylphényl.

4. Composé précurseur de colorant conforme à la revendication 1 qui a l'une des formules suivantes:

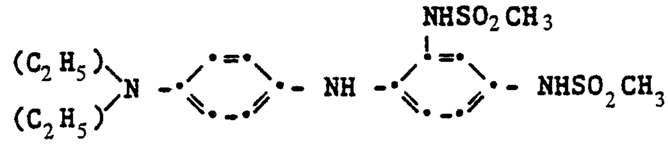

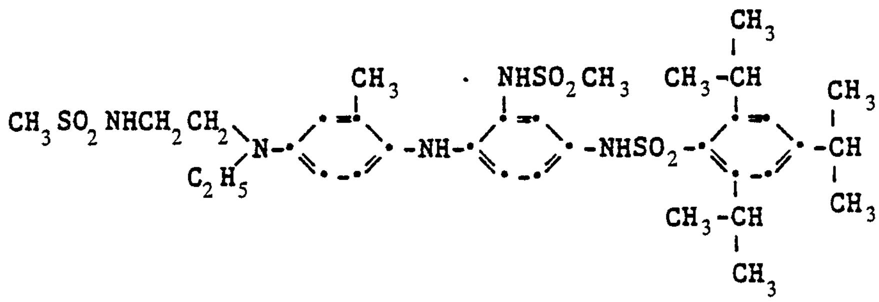

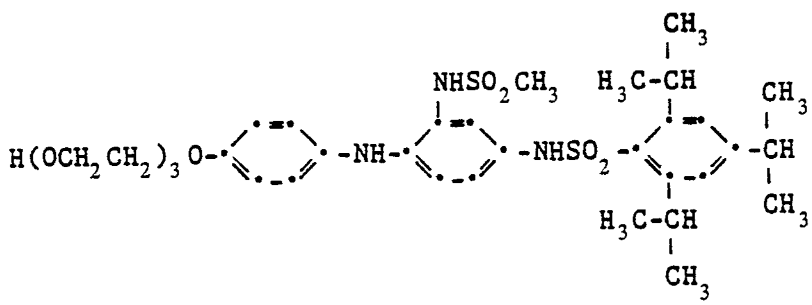

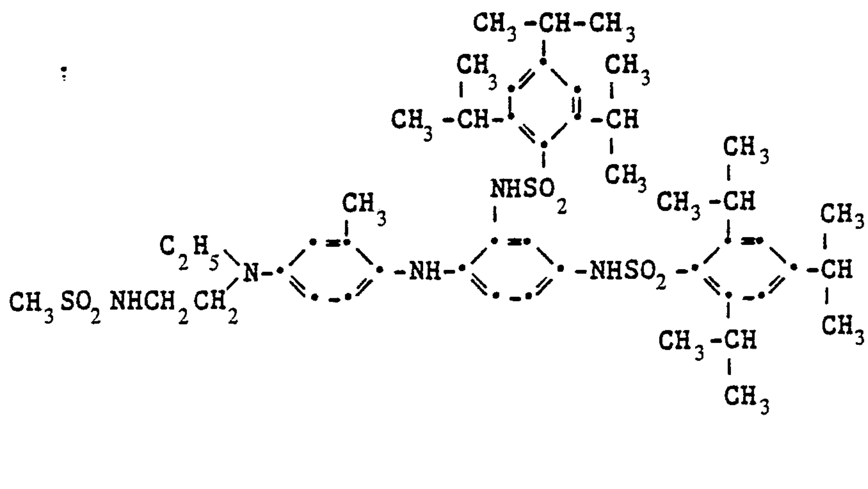

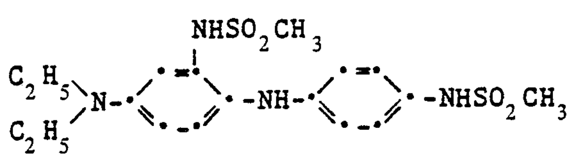

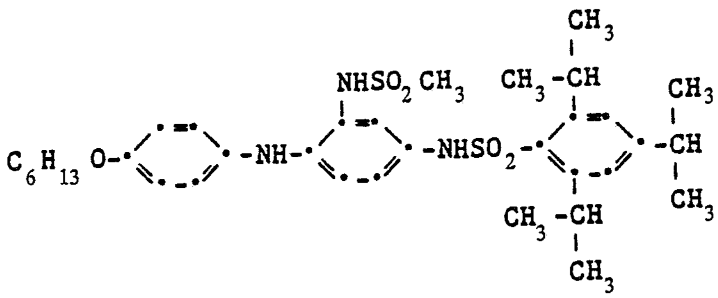

ou

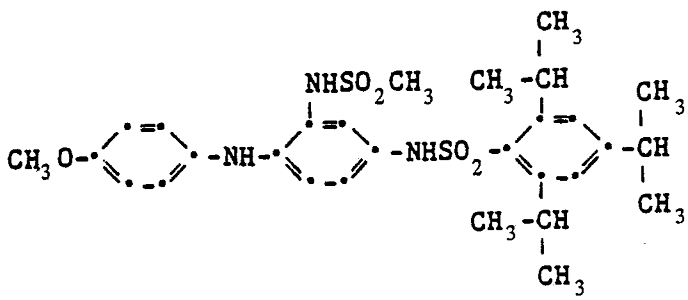

5. Produit photographique comprenant un support portant, en association réactive, un sel d'argent photographique et un composé précurseur de colorant conforme à l'une quelconque des revendications 1 à 4.

6. Produit photographique conforme à la revendication 5 comprenant, en outre un liant.

7. Produit photographique conforme à la revendication 5 ou 6 dans lequel le dit sel d'argent photographique consiste essentiellement en halogénures d'argent photographiques.

8. Produit photographique conforme à l'une quelconque des revendications 5 à 7 contenant un développateur des halogénures d'argent photographiques fonctionnant par oxydation croisée.

9. Produit photographique conforme à la revendication 8 dans lequel le développateur des halogénures d'argent photographiques est une 3-pyrazolidone.

10. Produit photographique conforme à l'une quelconque des revendications 6 à 9 dans lequel le liant est la gélatine.

11. Colorant phénazine comprenant un groupe sulfonamido en position 2.

12. Colorant phénazine conforme à la revendication 11 qui comprend, en outre, un groupe alkoxy, amino, mono- ou di-alkylamino en position 8.

13. Colorant phénazine conforme à la revendication 11 ou 12, représenté par la formule:

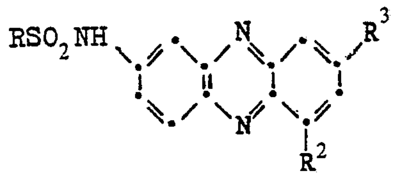

où:

R, $R^2$ et $R^3$ sont tels que définis à la revendication 2.

14. Colorant phénazine conforme à la revendication 13, dans lequel R est 2,4,6-triisopropylphényle.

15. Colorant phénazine conforme à la revendication 11 ou 13, qui correspond à l'une des formules suivantes:

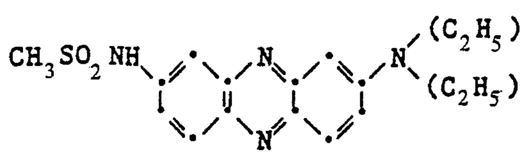

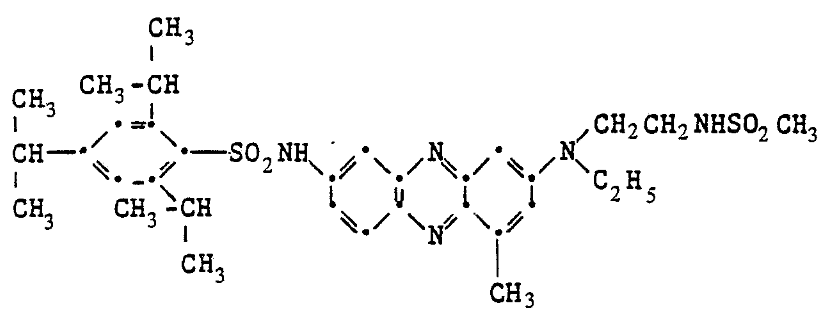

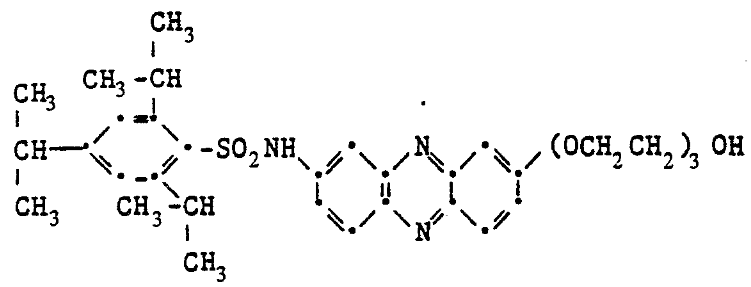

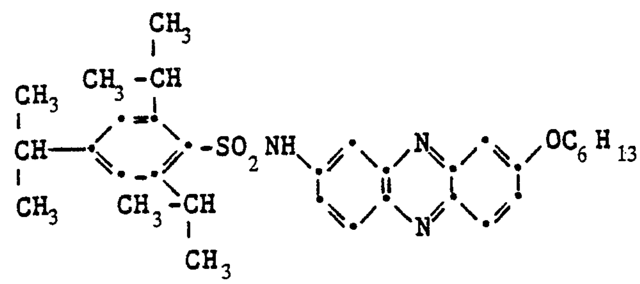

ou

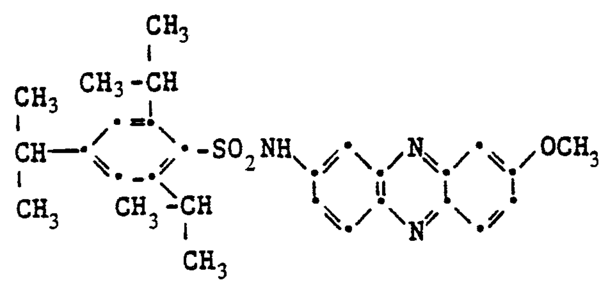

**Patentansprüche**

1. Farbbildende Sulfonamidodiphenylamin-Farbstoffvorläuferverbindung mit Sulfonamidogruppen in para-Stellung und in einer oder zwei der ortho-Stellungen bezüglich des —NH-Restes des Sulfonamidodiphenylamins, wobei das Sulfonamidodiphenylamin in oxidierter Form dazu befähigt ist, intramolekular unter Bildung eines Sulfonamido-substituierten Phenazin-Farbstoffes zu reagieren.

2. Farbstoffvorläuferverbindung nach Anspruch 1, gekennzeichnet durch die folgende Formel:

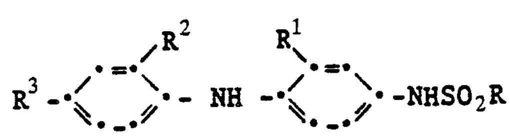

in der bedeuten:

R eine Alkylgruppe mit 1 bis 20 C-Atomen oder eine Arylgruppe mit 6 bis 20 C-Atomen;

$R^1$ und $R^2$ einzeln Wasserstoffatome, Alkylgruppen mit 1 bis 4 C-Atomen oder Reste der Formel $—NHSO_2R^4$, wobei gilt, daß mindestens einer der Substituenten $R^1$ und $R^2$ für einen Rest der Formel $—NHSO_2R^4$ steht;

$R^3$ eine Alkoxygruppe mit 1 bis 20 C-Atomen, eine Alkylgruppe mit 1 bis 20 C-Atomen oder eine Gruppe der Formel

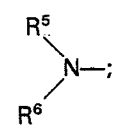

$R^4$ eine Alkylgruppe mit 1 bis 20 C-Atomen oder eine Arylgruppe mit 6 bis 20 C-Atomen;

$R^5$ ein Wasserstoffatom oder eine Alkylgruppe mit 1 bis 20 C-Atomen und

$R^6$ eine Alkylgruppe mit 1 bis 20 C-Atomen, wobei gilt, daß sämtliche Alkl-, Aryl- und Alkoxygruppe gegebenenfalls substituiert sein können.

3. Farbstoffvorläuferverbindung nach Anspruch 2, dadurch gekennzeichnet, daß R für eine 2,4,6-Triisopropylphenylgruppe steht.

4. Farbstoffvorläuferverbindung nach Anspruch 1, dadurch gekennzeichnet, daß sie einer der folgenden Formeln entspricht:

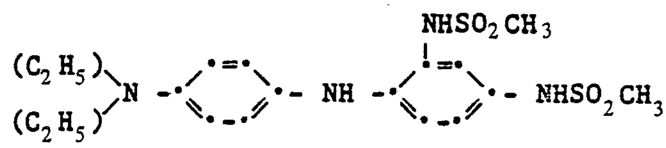

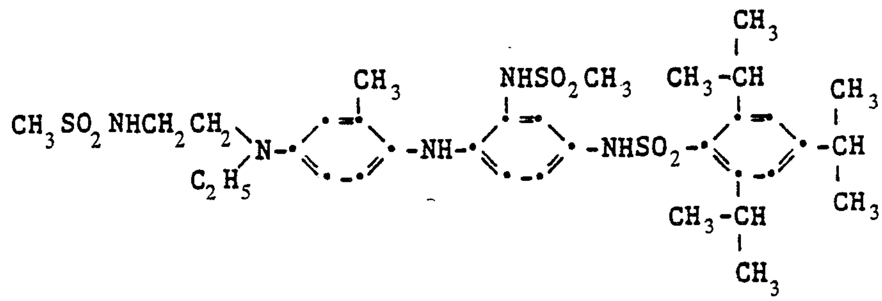

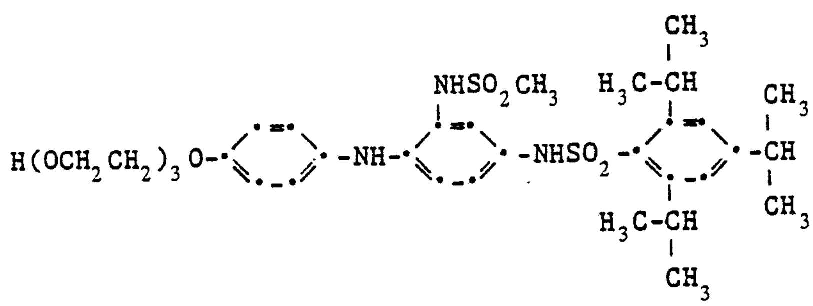

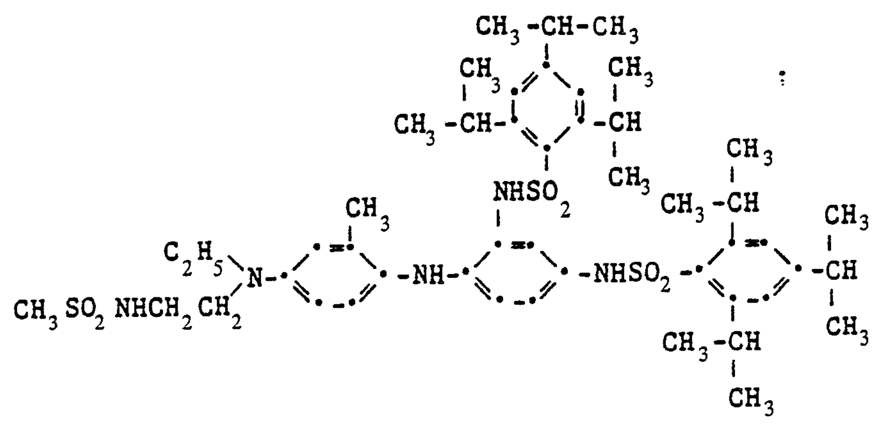

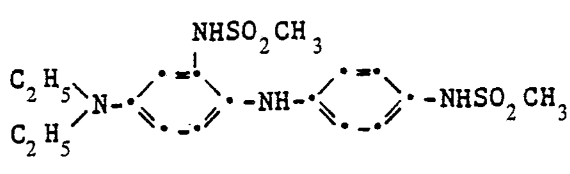

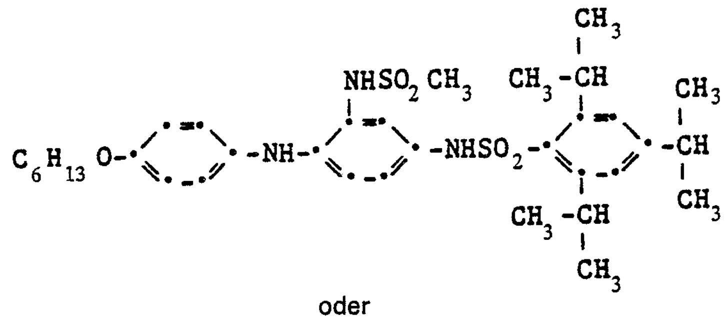

oder

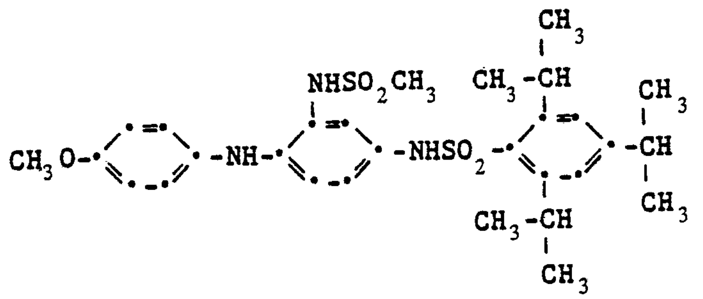

5. Photographisches Element mit einem Schichtträger, auf dem in reaktionsfähiger Vereinigung ein photographisches Silbersalz und eine Farbstoffvorläuferverbindung nach einem der Ansprüche 1 bis 4 vorliegen.

6. Photographisches Element nach Anspruch 5, dadurch gekennzeichnet, daß es ferner ein Bindemittel enthält.

7. Photographisches Element nach Anspruch 5 oder 6, dadurch gekennzeichnet, daß das photographische Silbersalz im wesentlichen aus photographischem Silberhalogenid besteht.

8. Photographisches Element nach einem der Ansprüche 5 bis 7, dadurch gekennzeichnet, daß es eine zu einer Überkreuz-Oxidation befähigte photographische Silberhalogenidentwicklerverbindung enthält.

9. Photographisches Element nach Anspruch 8, dadurch gekennzeichnet, daß die photographische Silberhalogenidentwicklerverbindung ein 3-Pyrazolidon ist.

10. Photographisches element nach einem der Ansprüche 6 bis 9, dadurch gekennzeichnet, daß das Bindemittel Gelatine ist.

11. Phenazinfarbstoff mit einer Sulfonamidogruppe in der 2-Stellung.

12. Phenazinfarbstoff nach Anspruch 11, dadurch gekennzeichnet, daß er des weiteren eine Alkoxy-, Amino-, Mono- oder Dialkylaminogruppe in der 8-Stellung aufweist.

13. Phenazinfarbstoff nach Anspruch 11 oder 12, gekennzeichnet durch folgende Formel:

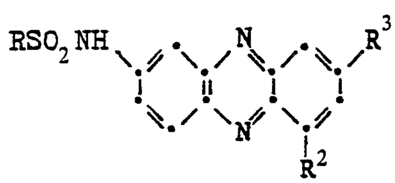

in der R, $R^2$ und $R^3$ die in Anspruch 2 angegebene Bedeutung haben.

14. Phenazinfarbstoff nach Anspruch 13, dadurch gekennzeichnet, daß R für eine 2,4,6-Triisopropylphenylgruppe steht.

15. Phenazinfarbstoff nach Anspruch 11 oder 13, gekennzeichnet durch eine der folgenden Formeln:

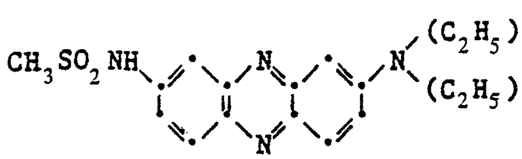

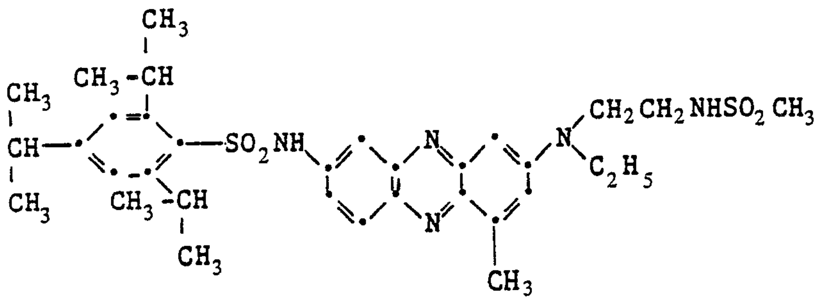

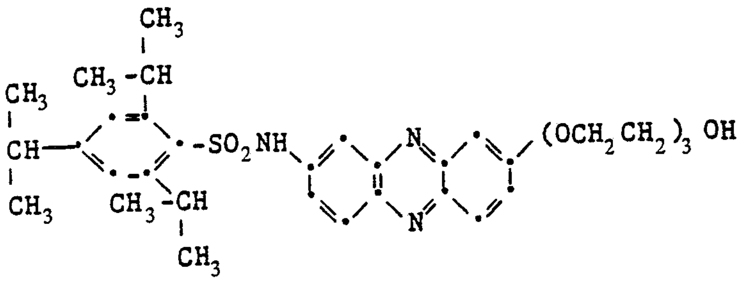

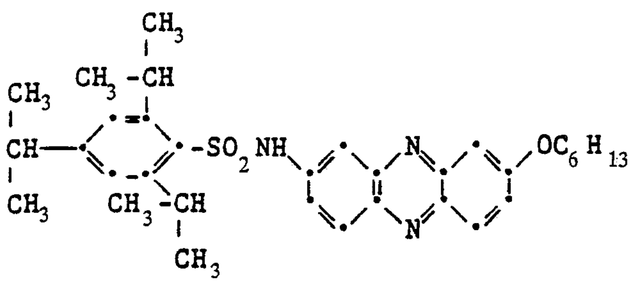

oder

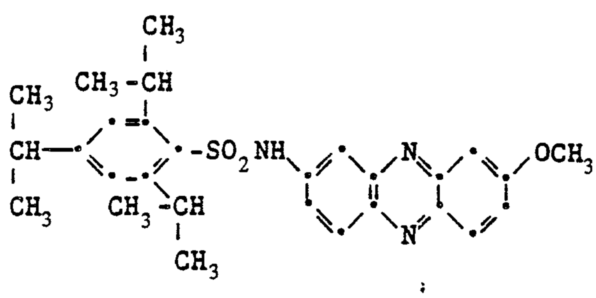

;